# EUROPEAN PATENT SPECIFICATION

(11) **EP 1 866 334 B1**
(45) Date of publication and mention of the grant of the patent: **02.10.2013**
(21) Application number: 05796484.3
(22) Date of filing: 05.10.2005
(51) Int. Cl.: C07K 14/57, C07K 14/56, C12N 15/62, A61K 38/21

(54) **INHIBITOR OF ENDOGENOUS HUMAN INTERFERON - GAMMA**
HEMMSTOFF VON ENDOGENEM MENSCHLICHEM INTERFERON GAMMA
INHIBITEUR DE L INTERFERON-GAMMA HUMAIN ENDOGENE

(30) Priority: 21.03.2005 BG 10908705
(43) Date of publication of application: 19.12.2007
(73) Proprietor: Ivanov, Ivan, 1517 Sofia (BG); Tsanev, Rumen, 1505 Sofia (BG); Nacheva, Genoveva, 1799 Sofia (BG); Grigoleit, Hans-Guenther, 65193 Wiesbaden (DE); Gunther, Rolf, 55124 Mainz (DE)
(72) Inventor: Ivanov, Ivan, 1517 Sofia (BG); Tsanev, Rumen, 1505 Sofia (BG); Nacheva, Genoveva, 1799 Sofia (BG); Grigoleit, Hans-Guenther, 65193 Wiesbaden (DE); Gunther, Rolf, 55124 Mainz (DE)
(74) Representative: Stefanova, Stanislava Hristova
(86) International application number: PCT/BG2005/000013
(87) International publication number: WO 2006/099701

(56) References cited:
- WO-A-00/43033
- WO-A-94/12531
- NACHEVA GENOVEVA ET AL: "Human interferon gamma: Significance of the C-terminal flexible domain for its biological activity." ARCHIVES OF BIOCHEMISTRY AND BIOPHYSICS, vol. 413, no. 1, 1 May 2003 (2003-05-01), pages 91-98, XP002373770 ISSN: 0003-9861

### SEQUENCE LISTING

<110> IVANOV, IVAN G.
   TSANEV, RUMEN G.
   NACHEVA, GENOVEVA A.
   GRIGOLEIT, HANS-GUENTHER
   GUNTHER, ROLF
<120> INHIBITOR OF ENDOGENOUS HUMAN INTERFERON- GAMMA
<130> stefanova-13
<140> PCT/BG2005/000013
   <141> 2005-10-05
<160> 4
<170> PatentIn version 3.3
<210> 1
   <211> 19
   <212> DNA
   <213> Artificial
<220>
   <223> synthetic forward primer for hIFN-gamma gene, introducing a HindIII site at the 5'-end
<400> 1
   cccaagctta tgcaggacc 19
<210> 2
   <211> 26
   <212> DNA
   <213> Artificial
<220>
   <223> synthetic reverse primer for introducing EcoRI site and to eliminate the last 27 codons from the 3'-end of hIFN-gamma gene
<400> 2
   ccggaattcc acttggatga gttcat 26
<210> 3
   <211> 21
   <212> DNA
   <213> Artificial
<220>
   <223> synthetic forward primer for modification the IFN-alpha gene
<400> 3
   ccggaattcg aggttgtcag a 21
<210> 4
   <211> 22
   <212> DNA
   <213> Artificial
<220>
   <223> synthetic reverse primer for introducing stop-codon and BamH1 cloning site at 3'-end of the IFN-alpha gene fragment
<400> 4
   cgcggatcct tattccttcc tc 22

## Description

### Field of invention

The invention relates to an inhibitor of endogenous human interferon-gamma (hIFN-γ), applicable for treatment autoimmune diseases, especially for multiple sclerosis.

### Background of invention

About 2% of the human population is affected by various autoimmune diseases, including multiple sclerosis (MS). MS is neurodegenerative disease affecting the central nervous system (CNS) and leading to a progressive physical disability. Although the exact etiology and pathogenesis of MS is still obscure, it is believed that it might be autoimmune disease [1]. Histopathology of MS is characterized with demyelination of motor neurons in CNS, loss of oligodendrocytes and moderate inflammatory reaction. Affected areas in the brain are usually infiltrated with T-lymphocytes and macrophages. T-lymphocytes belong to the CD+ subtype and are characterized with increased production of Th1 cytokines (IL-2 and IFN-γ) [2]. As a result, the mononuclear cells are induced to produce increased amounts of some destructive substances such as lymphotoxines (LT) and tumor necrosis factor alpha (TNF-α). Many studies show that the abnormal production of IFN-γ plays a key role in the pathogenesis of MS [3-6].

Recombinant DNA technology reveals new approaches for neutralizing the activity of endogenous hIFN-γ to find application for treatment of autoimmune diseases including MS. An inhibitor of the hIFN-γ secretion is hIFN-β, which has already been applied for treatment of MS patients [Patents US082138, WO9530435, CA2361081]. Patents RU2073522, RU2187332, RU02166959 recommend treatment with a mixture of hIFN-α, hIFN-β and hIFN-γ. It is reported, however, that the high daily doses of hIFN-β (8x10⁶ IU) results in unfavorable consequences related with the following effects of hIFN-β: a) hIFN-β blocks the T-cells proliferation [7]; b) hIFN-β neutralizes IL-12 thus enhancing the effect of hIFN-γ on dendrite cells [8]; hIFN-β suppresses the activity of T cells, producing hIFN-γ and IL-4, thus lowering the level of CD4+ cells (Th1, Th2) and CD8+ (Tc1) cells without changing the ratio Th1/Th2 [9, 10]; d) after a short-term treatment of MS patients during the acute phase hIFN-β decreases the expression of pro-inflamatory cytokines (such as hIFN-γ and hIFN-α) and increases the expression of anti-inflamatory cytokines (IL-4 and IL-10) [11].

Another approach for healing MS patients consists in neutralizing the endogenous hIFN-γ by specific monoclonal antibodies [12, 13, W00145747]. The long-term treatment with anti-hIFN-γ antibodies, however, results in deterioration of the health conditions, probably because of weakening of the natural defense system.

Patents US0086534 and CA2299361 offer a different approach for suppressing the abnormal production of IFN-γ based on the so called consensus interferons (IFN-con₁, IFN-con₂ and IFN-con₃) belonging to the groups of hIFN-α, hIFN-β and hIFN-τ. These recombinant preparations, however, show side effects, including toxicity.

Proteins with aminoacid sequence partly coinciding with that of the hIFN-γ have been applied as antiviral, antitumor and immunomodulating agents [US4832959, WO0208107, AT393690]. Their effects, however, is hard to be assessed since the descriptions are not supported with experimental data.

It is known, that there are at least two obligatory consecutive steps for hIFN-γ to express its biological activity: 1) to bind to its receptor and 2) to trigger the signal transduction cascade inside the cell; if a derivative molecule is not active, this might mean either that it is incapable of binding to the hIFN-γ receptor (i.e. lost affinity) or incapable of triggering the signal transduction pathway.

The paper of G. Nacheva at al. [15] reveals that a truncation of more than 21 C- terminal amino acids leads to a complete loss of hIFN-γ activity. But the authors do not ascertain that any of their nine inactive variants of hIFN-γ is a competitor of the native hIFN-γ for its receptor.

Subramaniam P. et al. [16] describe another 23 C-terminally deleted variant of the hIFN-γ, which is biologically inactive, but is still able to bind to the hIFN-γ receptor complex. The paper does not give any information regarding applicability of their investigation in medical practice.

### Description

The invention relates to an inhibitor of endogenous human interferon-gamma (hIFN-γ) in autoimmune diseases, especially in multiple sclerosis. More precisely, the invention relates to inactivated protein derivatives of the hIFN-γ with preserved affinity to the hIFN-γ receptor. These inactivated protein derivatives of the hIFN-γ represent a hybrid protein where the C-terminal part of the hIFNγ corresponds to that of the hIFN-α or is a recombinant hIFNγ, irradiated with UV light at 290 nm.

The inactivated protein derivatives of the hIFN-γ according to the invention are constructed on the basis of both the spatial structure and functional map of hIFN-γ. Since the receptor binding sites are located in the N-terminal region, the primary structures of the inactivated protein derivatives according to the invention coincides with that part of the hIFN-γ molecule.

### 1. Genetically modified variants of hIFN-γ where the C-terminal part of the molecule is replaced with a polypeptide sequence of another human protein (Hybrid hIFN-γ/ hIFN-α protein)

The genetically modified variants of hIFN-γ where the C-terminal part of the molecule is substituted, represent a hybrid molecule where 27 aminoacids originating from a human proteins such as IFN-α, IFN-β, IL-2, etc. are substituted at the C-terminal part of the human IFN-γ. The size of the hybrid protein is 143 aminoacid residues (equal to that of the human IFN-y). The hybrid IFN-γ/IFN-α gene is constructed by ligation of two DNA molecules one of which (containing 116 codons) originates from the 5'-terminal part of the hIFN-γ gene and the other (containing 27 in frame codons) comes from the 3'-terminal part of the IFN-α gene. The two gene fragments are prepared by PCR using full size hIFN-γ and hIFN-α genes as templates and a set of four synthetic primers. The forward primer for the hIFN-γ gene (SEQ ID No: 1) is designed to introduce a *Hind*III site at the 5'-terminus and the reverse primer (SEQ ID No: 2) to introduce a *Eco*RI site and also to eliminate the last 27 codons from the 3'-terminus of the hIFN-γ gene. The forward primer designed for modification of the IFN-α gene (SEQ ID No: 3) introduces an *Eco*RI site at the 5'-terminus of the IFN-α gene fragment and also to remove all but the last 27 codons from the IFN-α gene. The reverse primer (SEQ ID No: 4) introduces a stop-codon (TAA) and a *Bam*H1 cloning site at the 3'-end of the IFN-α gene fragment. The two gene fragments are amplified by PCR, purified by agarose gel electrophoresis and ligated to each other and then to the expression vector pJP₁R₃. The expression plasmid thus obtained (containing the hybrid hIFN-γ/hIFN-α gene) is transformed into *E*. *coli* LE392 cells. Bacteria are cultivated and the hybrid protein is purified as described above. The antiviral test shows that the hybrid hIFN-γ/hIFN-α protein is devoid of antiviral activity on WISH cells and competes successfully with the intact hIFN-γ for the hIFN-γ receptor.

### 2. hIFN-γ inactivated by irradiation with UV light (Photoinactivated hIFN-γ)

hIFN-γ contains single tryptophan (Trp) residue, which is indispensable for its biological activity. This residue is destroyed as follows: Recombinant IFN-γ is irradiated with UV light at 290 nm for 15 min. The results show that the biological activity of the photoinactivated hIFN-γ decreases drastically and the inactivated protein competes successfully with the intact hIFN-γ for its receptor.

Biological tests with the three derivative compounds of the hIFN-γ according to the invention show undoubtedly that they all have their basic biological activities (antiviral and antiproliferative) lost or drastically decreased and also that they all compete with hIFN-γ for the hIFN-γ receptor. Due to these properties, the inactive hIFN-γ derivative compounds can be used for suppression of the endogenous hIFN-γ activity. Since this effect is dose dependent, the activity of the endogenous hIFN-γ can be modulated by varying blood concentration of the hIFN-γ derivative proteins. This approach is applicable in the cases when the overproduction of endogenous hIFN-γ causes health problems as in the case of autoimmune diseases, including MS.

### Brief description of the Figures

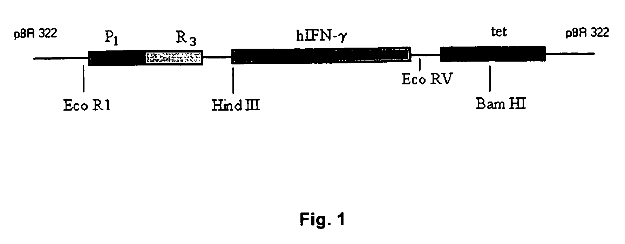

**Fig. 1** represents vector for expression of the hIFN-γ derivative, where:
   **P₁** is a synthetic phage promoter
   **R₃** is a synthetic ribosome binding site.
**Fig. 2** representing the inhibition of antiviral activity of the inactivated protein derivatives of recombinant hIFNγ

The following examples illustrate the present invention without limiting its scope and spirit:

### Example 1: Construction of a hybrid IFN-γ/IFN-α protein

The hybrid protein hIFN-γ/hIFN-α comprising 143 aminoacid residues consists of two N- and C-terminal parts: hIFN-γ (composed of 116 aminoacids) and hIFN-α (composed of 27 aminoacids). This protein is product of a hybrid hIFN-γ/hIFN-α gene prepared by ligation of two DNA fragments containing 116 (5' terminal) hIFN-γ and 27 (3' terminal) hIFN-α codons respectively. The two DNA molecules are obtained by PCR using full size hIFN-γ and hIFN-α genes as templates and two sets of primers (SEQ ID No 1-4). The forward primer for modification of the hIFN-γ gene (SEQ ID No: 1) is designed to introduce a *Hind*III site at the 5' terminus (for ligation to the expression vector) and the reverse primer (SEQ ID No: 2) introduces *Eco*RI site at the 3' terminus (for ligation to the hIFN-α gene). The latter is designed also to eliminate the last 27 codons from the hIFN-γ gene. The forward primer for the hIFN-α gene (SEQ ID No: 3) carries a *Eco*RI site at the 5' terminus (for ligation to the hIFN-γ gene) and also to removes all but the last 27 codons from the hIFN-α gene. The reverse primer (SEQ ID No: 4) is designed to introduce a stop-codon (TAA) and a *Bam*H1 site (for ligation to the expression vector) at the 3' end of the hIFN-α gene fragment.
PCR is carried out under conditions described in Tables 1 and 2 as follows:

**Table 1: Conditions for PCR**

| Programme | Number of cycles | Time | Temperature |
|---|---|---|---|
| I | 1 | 5 min | 92°C |
| II | 5 | 1min | 92°C |
| | | 1min | 60°C |
| | | 1min | 72°C |
| III | 35 | 1min | 92°C |
| | | 1min | 65°C |
| | | 1min | 72°C |
| IV | 1 | 10 min | 72°C |

**Table 2: Composition of the reaction mixture**

| Substances | Quantity |
|---|---|
| Tamplate DNA (50pg/µl) | 1µl |
| Reverse primer (20pmol/µl) | 1µl |
| Forward primer (20pmol/µl) | 1µl |
| Taq-polymeraze (3 U/µl) | 1µl |
| 10 x PCR buffer | 2µl |
| 2 mM dNTP's | 2µl |
| dH₂O | 12µl |
| Total | 20µl |

The amplified DNA fragments are digested with *Hind*III and *Eco*RI for hIFN-y and *Eco*RI and *Bam*HI for hIFN-α respectively. The DNA fragments are further purified by agarose gel electrophoresis and ligated first to each other and then to the expression vector. The expression plasmid carrying the hybrid hIFN-γ/hIFN-α gene is transformed into *E. coli* LE392 cells. Bacteria are cultivated and the hybrid protein is purified as described by two step chromatography on C8- Sepharose and CM- Sepharose as described in EP0446582. Antiviral activity (in international units) is determined by the protective effect of IFN-γ on WISH cells against the cytopatic action of stomatitis vesicular virus (VSV) as recommended by Forti et al. [14]. The antiviral test shows that the hybrid protein is devoid of any antiviral activity.

### Example 2: Inactivation of hIFN-γ by UV irradiation

Recombinant human hIFN-γ (purity higher than 99 %) is dissolved in 0.14 M NaCl, 10 mM Tris, pH 7.4 and exposed in a quartz cuvette to UV light at 290 nm for 15 min. This treatment leads to photolysis of the unique tryptophan residue and to 100 fold decrease in the hIFN-γ antiviral activity.

### Example 3: Inhibitory effect of inactive hIFN-γ derivative proteins on the biological activity of intact hIFN-γ

Inhibitory effect of inactive hIFN-γ derivative proteins on the biological activity of intact hIFN-γ is investigated using an amniotic cell line WISH (known to be rich of hIFN-γ receptors). To saturate the hIFN-γ receptors, WISH cells are pre-incubated with inactive hIFN-γ derivative proteins for 1 h. The proteins are washed out, the cells are treated with different concentrations of intact hIFN-γ and infected with VSV according to [14]. The obtained results are illustrated in Fig 2, where *a*) *human serum albumin is used as a neutral protein (depicted as blue diamonds); b) hIFNγ irradiated by UV light (depicted as squares); c) hIFNγ*/ *hIFNα hybrid protein (depicted as triangles);*

Since all hIFN-γ inactive derivative proteins preserve their affinity to the hIFN-γ receptor, they are capable of inhibiting the biological activity of the endogenous (native) hIFN-γ.

### REFERENCES

1. Waksman, B.H. and Reynolds W.E. (1984) Multiple sclerosis as a disease of immune regulation. Proc. Soc. Exp. Biol. Med., 175, 282-294
2. Oto, A.S., Guarion, T.J., Driver, R., Steinman, L., Umetsu, D.T. (1996) Regulation of disease susceptibility: decreased prevalence of IgE-mediated allergic disease in patients with multiple sclerosis. J. Allergy Clin. Immunol. 97, 1402-8
3. Johnson, K.P. (1988) Treatment of multiple sclerosis with various interferons: The cons. Neurology, 38 (suppl. 2) 52-64
4. Martino, G., Moiola, L., Brambilla, E., Clementi, E., Comi, G., Grimaldi, L.M. (1995). Interferon gamma induces T lymphocyte proliferation in multiple sclerosis via a Ca2+-dependent mechanism. J. Neuroimmunol. 62, 169-76
5. Vartanian, V., Li, Y., Zhao, M., Stefansson, K. (1995) Interferon-gamma-induced oligodendrocyte cell death: implications for the pathogenesis of multiple sclerosis. Mol. Med. 1, 732-43
6. Beck, J., Rondot, P., Catinot, L., Falcoff, E., Kirchner, H., Wietzerbin, J. (1988) Increased production of interferon gamma and tumor necrosis factor precedes clinical manifestation in multiple sclerosis: do cytokines trigger off exacerbations? Acta Neurol. Scand. 78, 318-323
7. Rep, M.H., Hintzen, R.Q., Polman, C.H., van-Lier, R.A. (1996) Recombinant interferon-beta blocks proliferation but enhances interleukin-10 secretion by activated human T-cells. J. Neuroimmunol. 67, 111-8
8. Heystek, H.C., den Drijver, B., Kapsenberg, M.L., van Lier, R.A., de Jong, E.C. (2003) Type I IFNs differentially modulate IL-12p70 production by human dendritic cells depending on the maturation status of the cells and counteract IFN-gamma-mediated signaling. Clin. Immunol. 107, 170-177
9. Franciotta, D., Zardini, E., Bergamaschi, R., Andreoni, L., Cosi, V. (2003) Interferon gamma and interleukin 4 producing T cells in peripheral blood of multiple sclerosis patients undergoing immunomodulatory treatment. J. Neurol. Neurosurg. Psychiatry. 74, 123-126
10. Furlan, R., Bergamim A., Lang, R., Brambilla, E., Franciotta, D., Martinelli, V., Comi, G., Paninam P., Martino. G. (2000) Interferon-beta treatment in multiple sclerosis patients decreases the number of circulating T cells producing interferon-gamma and interleukin-4.J. Neuroimmunol. 111, 86-92
11. Khademi, M., Wallstrom, E., Andersson, M., Piehl, F., Di Marco, R., Olsson,T. (2000) Reduction of both pro- and anti-inflammatory cytokines after 6 months of interferon beta-1a treatment of multiple sclerosis. J. Neuroimmunol. 103, 202-210
12. Skurkovich, S., Boiko, A., Beliaeva, I., Buglak, A., Alekseeva, T., Smirnova, N., Kulakova, O., Tchechonin, V., Gurova, O., Deomina, T., Favorova, O.O., Skurkovic, B., Gusev, E. (2001) Randomized study of antibodies to IFN-gamma and TNF-alpha in secondary progressive multiple sclerosis. Mult. Scler. 7, 277-284
13. Skurkovich, B., Skurkovich, S. (2003) Anti-interferon-gamma antibodies in the treatment of autoimmune diseases. Curr. Opin. Mol. Ther. 5, 52-57
14. Forti, R. L., Schuffman, S. S., Davies, H. A. and Mitchell, W. M. (1986) Objective antiviral assay of the interferons by computer assisted data collection and analysis. Methods in Enzymol. 119, 533-540
15. Nacheva, G., Todorova, K., Boycheva, M., Berzal-Herranz, A., Karshikov, A. and Ivanov, I. (2003) Human interferon-gamma: Significance of the C-terminal flexible domain for its biological activity. Arch. Biochem. Biophys. 413, 91-98
16. Subramaniam, P.S., Larkin, J. III, Mujtaba, M.G., Walter, M.R., Johnson, H.M. (2000) The COOH-terminal nuclear localization sequence of interferon gamma regulates STAT1 alpha nuclear translocation at an intracellular site. Journal of Cell Science 113, 2771-2781

## Claims

1. Inhibitors of endogenous hIFNγ based on inactivated protein derivatives of recombinant hIFNγ, **characterized in that** said inhibitor is an hIFNγ hybrid protein wherein the C-terminal part of the hIFNγ corresponds to that of the hIFN-α or said inhibitor is a recombinant hIFNγ, irradiated with UV light at 290 nm.

2. Use of inhibitors of the endogenous hIFNγ according to Claim 1 in the manufacture of a medicament for treatment of autoimmune diseases.

3. Use of inhibitors of the endogenous hIFNγ according to Claims 1 and 2 in the manufacture of a medicament for treatment of multiple sclerosis.

## Patentansprüche

1. Inhibitoren des endogenen hIFN-γ basierend auf inaktivierten Derivaten des rekombinanten hIFN-γ, **dadurch gekennzeichnet, dass** der genannte Inhibitor ein hybrides hIFN-γ Protein ist, wobei der C-terminale Abschnitt des hIFN-γ korrespondiert zum dem des hIFN-α oder der genannte Inhibitor ist ein rekombinantes hIFN-γ, welches mit UV-Licht der Wellenlänge 290 nm bestrahlt wurde.

2. Verwendung der Inhibitoren des endogenen hIFN-γ entsprechend Anspruch 1 bei der Herstellung als Medikament zur Behandlung von Autoimmunerkrankungen.

3. Verwendung der Inhibitoren des endogenen hIFN-γ entsprechend Ansprüchen 1 und 2 bei der Herstellung als Medikament zur Behandlung der Multiplen Sklerose.

## Revendications

1. Inhibiteurs de l'IFN-γ humain endogène basés sur des dérivés de protéines inactivés d'IFN-γ humain recombiné, **caractérisé en ce que** ledit inhibiteur est une protéine hybride d'IFN-γ humain où la section terminale C de l'IFN-γ humain correspond à celle de l'IFN-α humain ou ledit inhibiteur est un IFN-γ humain recombiné irradié par une lumière UV de 290 nm.

2. Utilisation d'inhibiteurs de l'IFN-γ humain endogène selon la revendication 1 dans la fabrication d'un médicament pour le traitement des maladies auto-immunes.

3. Utilisation d'inhibiteurs de l'IFN-γ humain endogène selon les revendications 1 et 2 dans la fabrication d'un médicament pour le traitement de la sclérose en plaques.
